Europäisches Patentamt

European Patent Office

Office européen des brevets

(19)

(11) Veröffentlichungsnummer: **0 229 972**
**B1**

(12) **EUROPÄISCHE PATENTSCHRIFT**

(45) Veröffentlichungstag der Patentschrift: **11.07.90**

(51) Int. Cl.⁵: **A 61 B 6/14**

(21) Anmeldenummer: **86117088.4**

(22) Anmeldetag: **08.12.86**

(54) Zahnärztiche Röntgendiagnostikeinrichtung zur Erstellung von Panorama-Schichtaufnahmen vom Kiefer eines Patienten.

(30) Priorität: **20.12.85 DE 3545493**

(43) Veröffentlichungstag der Anmeldung:
**29.07.87 Patentblatt 87/31**

(45) Bekanntmachung des Hinweises auf die
Patenterteilung:
**11.07.90 Patentblatt 90/28**

(84) Benannte Vertragsstaaten:
**DE FR GB IT**

(56) Entgegenhaltungen:
**EP-A-0 001 640**
**EP-A-0 035 307**
**DE-A-2 461 441**
**DE-A-2 630 135**
**DE-A-3 125 243**
**DE-A-3 503 465**
**DE-B-2 447 075**
**US-A-2 434 827**
**US-A-4 259 583**

(73) Patentinhaber: **Siemens Aktiengesellschaft**
**Wittelsbacherplatz 2**
**D-8000 München 2 (DE)**

(72) Erfinder: **Heubeck, Erich**
**Blütenweg 11**
**D-6140 Bensheim (DE)**
Erfinder: **Günther, Werner**
**Odenwaldstrasse 20**
**D-6140 Bensheim (DE)**
Erfinder: **Müther, Manfred**
**Adolf-Kolping-Strasse 20**
**D-6140 Bensheim (DE)**
Erfinder: **Werner, Leonhard, Dipl.-Ing.(FH)**
**Gleiwitzerstrasse 3**
**D-6944 Hemsbach (DE)**

**Beschreibung**

Die Erfindung bezieht sich auf eine zahnärztliche Röntgendiagnostikeinrichtung zur Erstellung von Panorama-Schichtaufnahmen vom Kiefer eines Patienten, mit einer Dreheinheit, welche einen Träger enthält für einerseits eine Strahlenquelle und andererseits einen die Filmkassette aufnehmenden Filmkassettenhalter, mit Verstelleinrichtungen, mittels welchen einerseits der Träger um vertikale Achslagerungen in einer dem Zahnbogen entsprechenden Umlaufkurve und andererseits der Filmkassettenhalter oder eine in ihm einlegbare Filmkassette so verstellbar sind, daß die Zähne aufeinanderfolgend mit dem Kiefer auf dem Film abgebildet werden, und mit wenigstens einer vor oder hinter der Filmkassette angeordneten Detektoranordnung, welche ein der Dosisleistung einer auf sie treffenden Strahlung entsprechendes elektrisches Signal zur Steuerung zumindest der Röhrenspannung (kV) der Strahlenquelle liefert.

Bei einer bekannten solchen Röntgendiagnostikeinrichtung (DE-A-24 47 075) wird der Kopf des Patienten in einer entsprechenden Halterungsvorrichtung gehalten. Während der Aufnahme bewegen sich Röntgenstrahlenquelle und Filmkassettenhalter um den Kopf des Patienten. Träger und Filmkassettenhalter werden dabei um vertikale Achsen so gedreht, daß die Röntgenstrahlen stets im wesentlichen rechtwinkelig auf den Kiefer bzw. die aufzunehmenden Zähne treffen, wobei der Abstand Zahn/Film im wesentlichen konstant bleibt. Infolge der Relativbewegung von Filmkassette und Strahlenquelle werden die Zähne aufeinanderfolgend mit dem Kiefer auf dem Film abgebildet.

Nach bisheriger Technik werden die Aufnahmedaten (kV, mA, sec), mit denen die Strahlenquelle betrieben wird, visuell vom Benutzer bestimmt und entsprechend vom Hersteller vorgegebenen Wertepaaren für kV/mA, die für verschiedene Körpergrößen (Kind, Jugendlicher, Erwachsener, Übergröße) empirisch ermittelt sind, in das Gerät eingegeben.

Es ist verständlich, daß eine solche, rein visuell getroffene Auswahl der Aufnahmedaten nicht immer optimal sein kann und eine sehr große Erfahrung des Gerätebenutzers voraussetzt. Weiterhin war es bei den bekannten Geräten nicht bzw. nur auf sehr umständliche Weise möglich, eine patientenbezogene Schichtlage zu ermitteln.

Der im Anspruch 1 angegebenen Erfindung liegt die Aufgabe zugrunde, demgegenüber eine Verbesserung zu erzielen und eine Röntgendiagnostikeinrichtung der eingangs genannten Gattung anzugeben, mit der eine einfachere und bessere Anpassung der Aufnahmedaten und Schichtlage an den Patienten erzielt werden kann, so daß einerseits eine optimale Filmschwärzung und andererseits eine geringere Patientenbelastung erreicht werden kann.

Vorteilhafte Ausgestaltungen und Weiterbildungen sind aus den Unteransprüchen zu entnehmen.

Nach dem erfindungsgemäßen Vorschlag wird zunächst im Rahmen der üblichen Durchstrahlung, die zunächst außerhalb des Kieferbogens beginnt, mittels der Detektoranordnung die ankommende Strahlendosis erfaßt und mit einem festgelegten, z.B. in einem Speicher abgelegten Dosiswert verglichen. Dieser Soll-Dosis-Wert repräsentiert den persönlichen Schwärzungsgrad, den der Arzt aufgrund des verwendeten Film- und Entwicklungsmaterials des gewählten Filmkassettenformates und gegebenenfalls noch weiterer Faktoren festlegt. Dieser Sollwert ist vorteilhafterweise entsprechend dem gewünschten Schwärzungsgrad einstellbar. Die vorgesehene Recheneinheit ermittelt aus dem Vergleich der Dosiswerte die für die Durchstrahlung des betreffenden Patientenkopfes erforderlichen Aufnahmedaten (mA, kV, sec). Mit den so ermittelten Aufnahmedaten erfolgt sodann die Durchstrahlung entsprechend der vorgegebenen Ablaufkurve.

Die für den betreffenden Patienten zu bestimmende optimale Schichtlage wird vorteilhafterweise dadurch ermittelt, daß die Zeit vom Beginn der Beaufschlagung der Filmkassette mit zunächst außerhalb des Kieferbogens verlaufenden Strahlen bis Erreichen der Sprungfunktion, d.h. bis zum Beginn der Durchstrahlung des Kiefers, gemessen und aus dieser Zeit zunächst die Kieferlänge (Abwicklung des Kiefers auf dem Film) und Kieferbreite (Kieferweite) ermittelt wird. Hierzu wird die der ermittelten Ablaufzeit bis Erreichen der Sprungfunktion zugehörige Strecke im Verhältnis zur Gesamtzeit, die notwendig ist, um den gesamten Kiefer aufzunehmen und der dazugehörigen Gesamtstrecke auf dem Film, ermittelt und aus der Gesamtstrecke abzüglich der Teilstrecke vor und nach der Durchstrahlung des Kiefers sodann die Kieferlänge errechnet. Aus der Kieferlänge wiederum kann dann mit Hilfe eines empirisch ermittelten Ümrechnungsfaktors die Kieferweite ermittelt werden. Aus dem Wert für die Kieferweite läßt sich entweder durch Vergleich mit vorgegebenen, beliebig fein abgestuften Sollwerten oder auch individuell errechnet die für den betreffenden Patienten, entsprechend seiner persönlichen Kieferkonstellation, vorzusehende optimale Filmgeschwindigkeit ermitteln. Mit der individuell ermittelten Filmgeschwindigkeit kann schließlich die optimale Schichtlage bestimmt werden; ein Vorteil, der mit den bekannten Geräten nicht zu erzielen ist.

Anhand der Zeichnung wird ein Ausführungsbeispiel der Erfindung näher erläutert. Es zeigen:

Figur 1 das erfindungsgemäße Gerät in einer schaubildlichen Darstellung,

Figur 2 eine vereinfachte Darstellung der Verstellmechanik für das Gerät nach Figur 1,

Figuren 3 und 4 schematische Darstellungen vom Kiefer eines Patienten einerseits in der Draufsicht und andererseits als Abbildung auf einen Film,

Figur 5 ein Blockschaltbild.

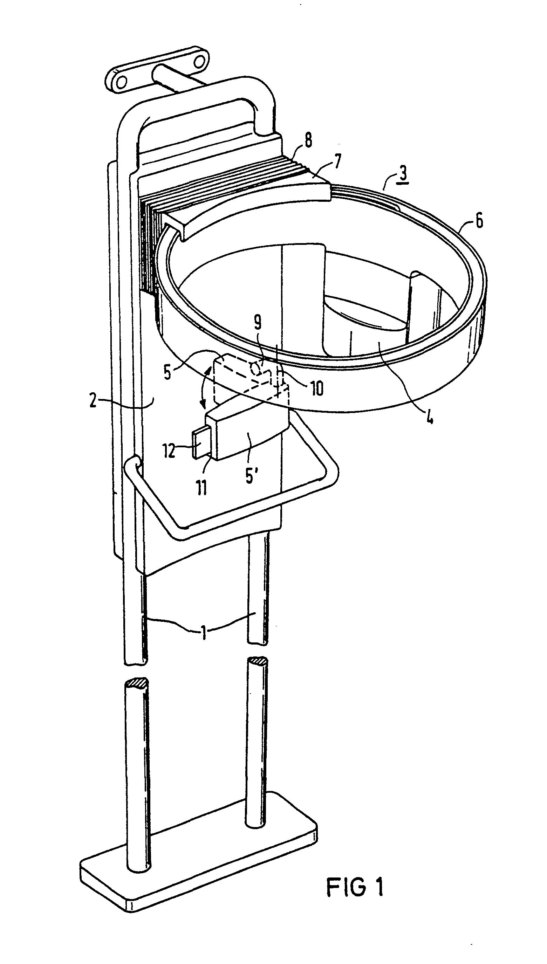

Die Figur 1 zeigt in einer schaubildlichen Darstellung eine mögliche Ausführungsform eines

2

Röntgendiagnostikgerätes nach der Erfindung. Das Gerät enthält ein aus zwei Standrohren gebildetes Stativ 1, an dem ein Laufwagen 2 höhenverstellbar angeordnet ist. Am Laufwagen 2 ist eine allgemein mit 3 bezeichnete Dreheinheit gehaltert, die einerseits eine Röntgenstrahlenquelle 4 und diametral dazu eine Filmkassettenhalterung 5 enthält. Träger für Röntgenstrahlenquelle 4 und Filmkassettenhalterung 5 ist ein in sich geschlossener Drehring 6, der in einer später noch erläuterten Weise in einem Lagerteil 7 dreh- und schwenkbar gehaltert ist. Die hierzu erforderliche Verstellmechanik ist durch einen zwischen Laufwagen 2 und Drehring 6 angeordneten Faltenbalg 8 abgedeckt.

Während die Strahlenquelle 4 am Drehring 6 fest angeordnet ist, ist die Filmkassettenhalterung 5 am freien Ende eines abgewinkelten, am Drehring 6 befestigten Tragarmes 9 um eine vertikale Achslagerung 10 schwenkbar gehaltert. Die Filmkassette 5 kann so zur besseren Patientenpositionierung bzw. für Spezialaufnahmen (Ceph-Aufnahmen) aus der in Figur gestrichelt eingezeichneten Gebrauchsposition in die mit durchgehenden Linien gezeichnete Nichtgebrauchsposition 5' gebracht werden. Die Filmkassettenhalterung 5 enthält an beiden Stirnseiten je einen Schlitz II; über den einen wird die mit 12 bezeichnete Filmkassette, welche den zu belichtenden Film enthält, eingeführt, über den anderen nach der Filmbelichtung ausgefahren.

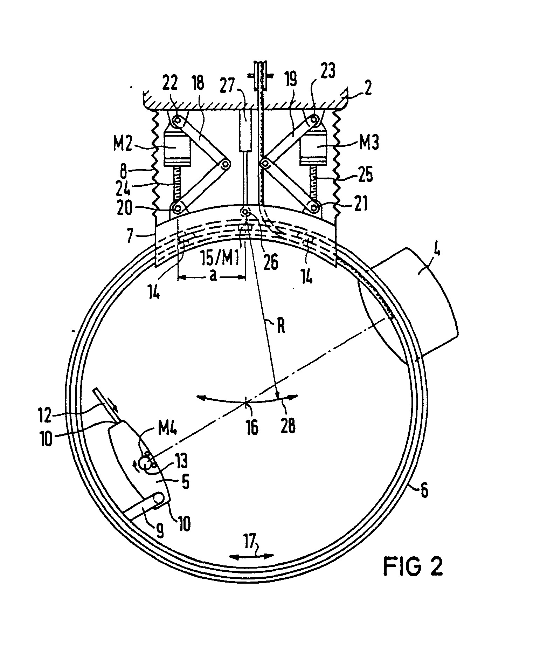

Die Filmkassette 12 wird von einer in Figur 2 allgemein mit 13 bezeichneten Transporteinrichtung, bestehend aus einem mit einer Antriebsrolle gekuppelten Antriebsmotor (M 4) und zwei korrespondierend dazu angeordneten Gegendruckrollen, mit einer bestimmten Geschwindigkeit an der Belichtungsstelle (Sekundärblende), an der der Zentralstrahl auftrifft, vorbeigeführt.

Der Drehring 6 ist im Lagerteil 7 drehbar gehaltert. Zu diesem Zweck sind unter Bildung einer Dreieckslagerung einerseits unterhalb des Drehringes im Lagerteil 7 zwei Führungsrollen 14 und andererseits mitig zwischen diesen beiden Führungsrollen 14 und oberhalb des Drehringes eine Antriebsrolle 15 vorgesehen. Die Antriebsrolle ist mit einem nicht näher dargestellten, allgemein mit M1 bezeichneten ersten Antriebsmotor gekuppelt. Über diesen, als Friktionsantrieb ausgebildeten Antrieb kann der Drehring um die mit 16 bezeichnete Mittelpunktsachse in Richtung der Pfeile 17 gedreht werden.

Zusätzlich zu dieser Eigendrehung ist noch eine Verstellung des Drehringes 6 in Relation zum Laufwagen 2 möglich. Hierzu sind zwei Scherenarme 18, 19 vorgesehen, deren eine Enden an den mit 20 und 21 bezeichneten Gelenkstellen am Lagerteil 7 und deren andere Enden an den mit 22 und 23 bezeichneten Gelenkstellen am Laufwagen 2 angelenkt sind. Zwischen den Gelenkstellen sind Spindelantriebe 24 und 25 vorgesehen, deren mit M2 und M3 bezeichnete Antriebsmotoren, wie später noch erläutert, individuell angesteuert werden können, wodurch der Drehring zusätzlich zu seiner Eigendrehung um die Achse 16 noch parallel oder schräg zum Laufwagen 2 verstellt werden kann. Hierzu ist mittig zwischen den beiden Gelenkstellen 20 und 21 in einem Abstand a das Lagerteil 7 mittels eines Schwenklagers 26 am einen Ende eines Teleskoparmes 27 angelenkt, dessen anderes Ende starr am Laufwagen 2 befestigt ist. In Verbindung mit der vorbeschriebenen Scherenarmkonstruktion ist es damit möglich, bei gleichmäßigem Antrieb beider Motoren M2, M3 den Drehring um die Schwenkachse 26 so zu verschwenken, daß der Mittelpunkt 26 des Drehringes 6 eine Querbewegung von etwa ± 40 mm mit dem Radius R von etwa 350 mm in der mit 28 angegebenen Pfeilrichtung ausführt. Der Bewegungsablauf des gesamten Systems kann so ohne aufwendige Mechanik auf relativ einfache Weise durch Steuern der einzelnen Motoren M1, M2, M3 sowie der Transporteinrichtung 13 für die Filmkassette erfolgen.

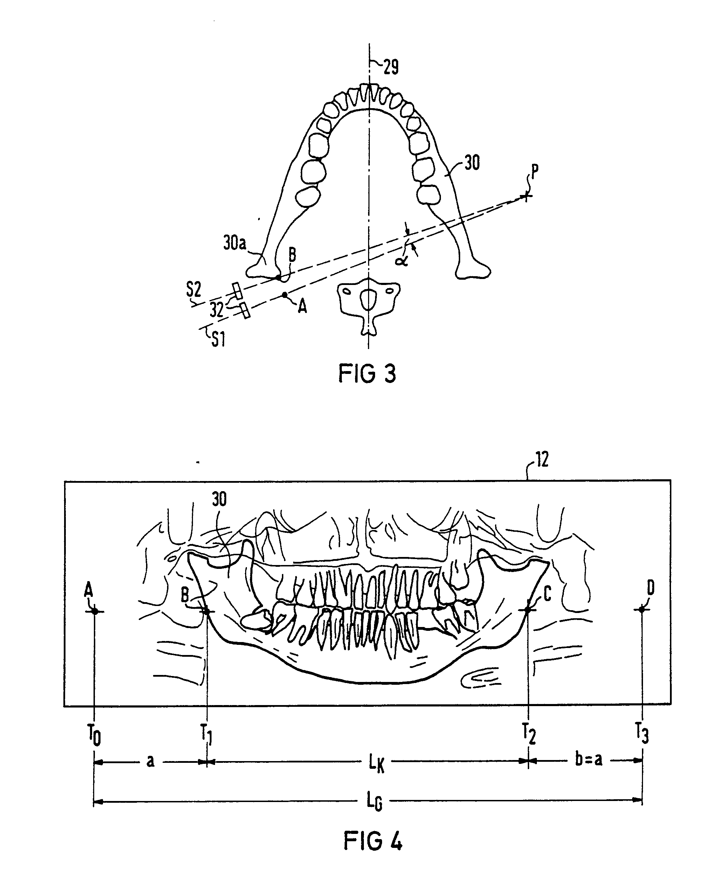

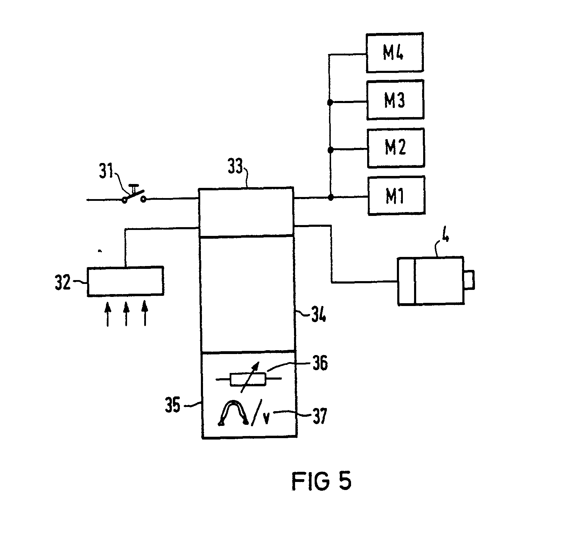

Anhand der Figuren 3 bis 5 wird die Aufnahmetechnik gemäß der Erfindung näher erläutert. Dabei ist in Figur 3 schematisch der Kiefer eines Patienten in Draufsicht dargestellt, in Figur 4 eine Panorama-Aufnahme vereinfacht dargestellt und in Figur 5 ein Übersichtsschaltbild angegeben.

Die Dreheinheit 3 (Fig. 1) wird zunächst in eine Ausgangsposition gefahren, von der aus die Strahlenquelle 4 von einem Punkt P aus (Fig. 3) den Kiefer durchstrahlen kann. Diese Ausgangsposition ist an sich beliebig wählbar, jedoch so zu legen, daß erste Strahlen S1 außerhalb des einen Endes des einen Kieferbogens 30 verlaufen, in der Darstellung z.B. außerhalb des linken Kieferbogens 30a. Eine vor oder hinter der Filmkassette angeordnete, aus ein oder mehreren Detektoren bestehende Detektoranordnung 32 erfaßt die von der Strahlenquelle abgegebene Strahlendosis und bildet in Abhängigkeit von der Strahlendosis elektrische Signale.

Nach Betätigen einer Starttaste 31 (Fig. 5) werden zum Zeitpunkt $T_0$ die Strahlenquelle 4 sowie die Antriebsmotoren M1 bis M3 für die Dreheinheit 3 und der Filmantrieb M4 für die Verstellung der Filmkassette 12 eingeschaltet. Die Verstellung der Dreheinheit erfolgt dabei mit konstanter Geschwindigkeit.

Angenommen, der Beginn der Bestrahlung liegt auf dem Film im Punkt A, so liegen diese Strahlen (S1 in Fig. 3) außerhalb des linken Kieferbogens 30a. Nach Beginn der Durchstrahlung des Patientenkopfes im Punkt A wird die auftreffende Strahlendosis mittels der Detektoranordnung 32 erfaßt. Diese gibt ein entsprechendes Signal an eine Steuereinheit 33. In einer Recheneinheit 34 mit Vergleichersehaltung wird eine der Ist-Strahlendosis entsprechende Größe mit einer in einem Speicher 35 abgelegten Soll-Größe, die einem für einen bestimmten Schwärzungsgrad des Filmes festgelegten Dosiswert entspricht, verglichen und daraus die zugehörigen Aufnahmedaten (ma, kV, sec) ermittelt. Die ermittelten Größen werden der Steuereinheit 33 zugeführt, welche den Generator der Strahlenquelle 4 steuert. Damit der Schwärzungsgrad individuell vom Benutzer bestimmbar ist, enthält der Speicher 35 einen einstellbaren Sollwert 36 für die Strahlendosis.

Nachdem bei manchen Patienten zu Beginn der Bestrahlung an der Ausgangsposition P die Wirbelsäule im Strahlengang liegen kann, ist in einem solchen Fall der von der Detektoranordnung 32 gemessene Dosiswert nicht zur Berechnung der Solldosis geeignet. Um diesbezügliche Verfälschungen zu vermeiden, wird, gemäß einer weiteren vorteilhaften Ausgestaltung der Erfindung vorgeschlagen, Mittel vorzusehen, die dazu führen, daß zu einem späteren Zeitpunkt, zu dem die Wirbelsäule in keinem Fall mehr im Strahlengang liegen kann, mindestens eine Wiederholungsmessung durchgeführt wird, und diese dann zur Berechnung der Solldosis herangezogen wird. Diese Mittel müssen einerseits einen Schwellwert definieren, unterhalb dem die von der Detektoranordnung 32 gemessene Dosis ignoriert wird und andererseits eine Zeit festlegen, nach der die Wiederholmessung ausgeführt wird.

Die Detektoranordnung besteht vorteilhafterweise aus Elementen, die aus einem Szintillationskristall und einer Fotodiode aufgebaut sind.

Neben der Ermittlung der patientenbezogenen Aufnahmedaten läßt sich mit der erfindungsgemäßen Anordnung auch eine patientenbezogene Schichtlage festlegen. Hierzu wird das Erreichen der Sprungfunktion, d.h. der Beginn der Durchstrahlung des linken Kieferbogens, nach der Wegstrecke a auf dem Film im Punkt B zum Zeitpunkt $T_1$ (Strahlen S2 in Fig. 3) mittels der Detektoranordnung 32 festgestellt, die in diesem Punkt eine Dosisschwächung registriert. Die Detektoranordnung 32 gibt nun ein weiteres Signal an die Steuereinheit 33. Zugleich wird die abgelaufene Zeit von $T_0$ bis $T_1$, welche von der Steuereinheit 33 erfaßt wird, der Recheneinheit 34 gemeldet. Die Recheneinheit 34 verarbeitet diese Information, indem sie aus der bekannten Laufzeit T ($T_0$ bis $T_3$), die notwendig ist, um den gesamten Kiefer aufzunehmen, und der ebenfalls bekannten Weglänge $L_G$ auf dem Film vom Beginn der Durchstrahlung im Punkt A bis zum Abschalten des Strahlers nach Beendigung der Aufnahme zum Zeitpunkt $T_3$ im Punkt D die Länge des Kiefers $L_K$ entsprechend seiner Abwicklng auf dem Film nach der Beziehung $L_K = L_G - 2 \cdot a$ ermittelt.

Hierbei wird zugrundegelegt, daß die Aufnahme symmetrisch zum Kieferbogen 30 und damit zur Kiefersymmetrieachse 30 erfolgt.

Die für den betreffenden Patienten zu bestimmende optimale Schichtlage kann durch Vergleich mit empirisch ermittelten und in einem Speicherteil 37 des Speichers 35 abgelegten Wertepaaren ($\Omega/v$) für bestimmte Kieferweiten zugeordneten Filmgeschwindigkeiten bestimmt werden. Die Recheneinheit 34 ermittelt also aus der patientenspezifischen Kieferkonstellation (Kieferlänge/Kieferweite) die dazugehörige Filmgeschwindigkeit, mit welcher über die Steuereinheit 33 der Filmantrieb M4 gesteuert wird.

## Patentansprüche

1. Zahnärztliche Röntgendiagnostikeinrichtung zur Erstellung von Panorama-Schichtaufnahmen vom Kiefer eines Patienten, mit einer Dreheinheit (3), welche einen Träger (6) enthält für einerseits eine Strahlenquelle (4) und andererseits einen eine Filmkassette (12) aufnehmenden Filmkassettenhalter (5), enthaltend ferner Verstelleinrichtungen (13, M1 bis M3), mit welchen einerseits der Träger (6) um vertikale Achslagerungen (16, 26) in einer dem Zahnbogen entsprechenden Umlaufkurve und andererseits der Filmkassettenhalter (5) oder eine in ihm einlegbare Filmkassette (12, 50) so verstellbar ist, daß die Zähne aufeinanderfolgend mit dem Kiefer auf dem Film abgebildet werden, und enthaltend ferner wenigstens eine vor oder hinter der Filmkassette (12) angeordnete Detektoranordnung (32), welche ein der Dosisleistung einer auf sie treffenden Röntgenstrahlung entsprechendes elektrisches Signal zur Steuerung zumindest der Röhrenspannung (kV) der Strahlenquelle (4) liefert, dadurch gekennzeichnet, daß die Dreheinheit (3) mittels der Verstelleinrichtungen (M1 bis M3) in eine Ausgangsposition (P) einstellbar ist, von der aus, unter Verstellung von Dreheinheit (3) und Filmkassette (12), entsprechend der Umlaufkurve, die Filmkassette (12) mit Strahlen (S1, S2) beaufschlagt wird, die außerhalb des einen Endes (30a) des Kieferbogens (30) verlaufen, daß mittels der Detektoranordnung (32) die ankommende Strahlendosis erfaßt wird, daß ein der Strahlendosis entsprechendes Signal einer Recheneinheit (34) zugeführt Wird, in der eine dem Signal entsprechende Größe mit einer einem vorgegebenen Dosiswert entsprechenden Größe verglichen Wird, und daß aus dem Vergleich die für die Durchstrahlung des betreffenden Patientenkopfes erforderlichen Aufnahmedaten (mA,kV,sec) ermittelt werden, mit denen der Generator der Strahlenquelle (4) gesteuert wird.

2. Röntgendiagnostikgerät nach Anspruch 1, dadurch gekennzeichnet, daß mit der Recheneinheit (34) ein Speicher (35) verbunden ist, der ein Speicherglied (36) enthält, welches die dem vorgegebenen Dosiswert entsprechende Vergleichsgröße liefert.

3. Röntgendiagnostikgerät nach Anspruch 2, dadurch gekennzeichnet, daß ein in seiner Vergleichsgröße einstellbares Speicherglied (36) vorgesehen ist.

4. Röntgendiagnostikgerät nach Anspruch 1, dadurch gekennzeichnet, daß aus der Dosismessung am Film mittels der Detektoranordnung (32) zunächst patientenspezifische Größen für Kieferlänge/Kieferweite ermittelt werden und die patientenspezifische Schichtlage aus einem Vergleich dieser Größen mit vorgegebenen Wertepaaren für bestimmten Kieferabmessungen zugeordneten Filmgeschwindigkeiten festgelegt wird.

5. Röntgendiagnostikgerät nach Anspruch 4, dadurch gekennzeichnet, daß die Zeit vom Beginn der Durchstrahlung des Patientenkopfes bis zum Beginn der Durchstrahlung des Kiefers von einer Steuereinheit (33) erfaßt wird, daß in der Recheneinheit (34) aus der ermittelten Zeit (T₁) im Verhältnis zur Gesamtlaufzeit (T) und der in dieser Zeit (T) auf dem Film (12) zurückgelegten Wegstrecke (L_G) zunächst die Wegstrecke (a) auf dem Film bis zum Beginn der Durchstrahlung des Kiefers, danach die Länge (L_K) des auf dem Film (12) abgebildeten Kiefers (30) und hieraus die Größe für die Kieferlänge (L_K) errechnet wird.

6. Röntgendiagnostikgerät nach Anspruch 5, dadurch gekennzeichnet, daß die Recheneinheit (34) einen Speicher (35) enthält oder mit einem solchen verbunden ist, in dem mehrere Vergleichswerte (37) für unterschiedlichen Kieferweiten zugeordnete Filmgeschwindigkeiten abgelegt sind, daß der von der Recheneinheit (34) ermittelte Wert für die patientenspezifische Kieferweite mit den abgelegten Speicherwerten verglichen und aus dem Vergleich die der persönlichen Kieferkonstellation des Patienten entsprechende Schichtlage bestimmt wird.

7. Röntgendiagnostikgerät nach einem der Ansprüche 1 bis 6, dadurch gekennzeichnet, daß die Verstelleinrichtung erste Verstellmittel (14, 15, M1) enthält, mit welchen der Träger (6) um eine erste vertikale Achslagerung (16) gedreht werden kann, daß zweite Verstellmittel (18 bis 25, M2, M3) vorhanden sind, mit welchen der Träger während der Drehbewegung um die erste Achslagerung eine Schwenkbewegung quer zur Symmetrieachse (30) des Objektes ausführen kann, wobei der Schwenkradius (R) und die Auslenkung (28) der Schwenkbewegung so gewählt sind, daß bei der Bewegung der Dreheinheit (6) stets eine senkrechte Durchstrahlrichtung durch das Objekt bei konstantem Abstand von Objekt und Film vorgesehen ist und daß ferner die Filmkassette (12) in ihrer Halterung (5) relativ zur Strahlenquelle (4) verstellbar gehaltert ist und daß eine Steuereinheit (33) zur Ansteuerung der Verstellantriebe (M1 bis M4) vorhanden ist.

8. Röntgendiagnostikgerät nach Anspruch 7, dadurch gekennzeichnet, daß der Träger als vorzugsweise geschlossener Drehring (6) ausgebildet ist, der an einem mit dem Laufwagen (2) verbundenen Lagerteil (7) drehbar gefaßt ist, daß die Filmkassette (12) in der am Ring (6) angeordneten Kassettenhalterung (5) relativ zur Strahlenquelle (4) verstellbar gehaltert ist, und daß die Verstelleinrichtung Mittel (14, 15, M1; 18 bis 25, M2, M3) zur Verstellung des Ringes (6) einerseits um seinen Mittelpunkt (16) und andererseits um eine am Lagerteil (7) angeordnete, mit dem Laufwagen (2) verbundene Schwenkachse (26) mit vertikaler Achslagerung enthält.

9. Röntgendiagnostikgerät nach Anspruch 8, dadurch gekennzeichnet, daß das Lagerteil (7) mittels zweier, beidseitig der Schwenkachse (26) in einem Abstand (a) angeordneter Scherenarme (24, 25) am Laufwagen (2) angelenkt ist und zwischen den Gelenkstellen (21, 23; 20, 22) wenigstens eines Scherenarmes (24, 25) die Verstellmittel (M2, M3) angeordnet sind.

10. Röntgendiagnostikgerät nach Anspruch 9, dadurch gekennzeichnet, daß zwischen den Gelenkstellen (20, 23) beider Scherenarme (24, 25) ein Spindelantrieb vorgesehen ist und an der Schwenkachse (26) das eine Ende eines Teleskoparmes (27) angelenkt ist, dessen anderes Ende am Laufwagen (2) starr befestigt ist.

11. Röntgendiagnostikgerät nach einem der Ansprüche 1 bis 3, dadurch gekennzeichnet, daß zur Berechnung des einen Sollwert repräsentierenden Dosiswertes Mittel vorhanden sind, welche einerseits einen Schwellwert definieren, unterhalb dem die von der Detektoranordnung (32) gemessene Dosis unberücksichtigt bleibt und welche andererseits eine Zeit festlegen, nach deren Ablauf eine Wiederholungsmessung ausgeführt wird.

12. Röntgendiagnostikgerät nach einem der Ansprüche 1 bis 11, dadurch gekennzeichnet, daß eine optoelektronische Detektoranordnung (32) vorgesehen ist, mit Elementen, die aus einem Szintillationskristall einerseits und einer Fotodiode andererseits aufgebaut sind.

**Revendications**

1. Installation de radiodiagnostic dentaire pour réaliser des tomographies panoramiques de la mâchoire d'un patient, comportant une unité d'entraînement en rotation (3) pourvue d'un support (6) pour, d'une part, une source de rayonnement (14) et pour, d'autre part, un support de cassette à film (12), comportant, en outre, des dispositifs de déplacement (13, M1 à M3) à l'aide desquels sont déplaçables de telle manière, d'une part, le support (6) autour de paliers axiaux verticaux (16, 26) suivant une courbe qui correspond à l'allure cintrée de la dentition, et, d'autre part, le support de cassette à film (5) ou une cassette à film (12, 50) susceptible d'être introduite dans ce dernier, que les dents sont reproduites successivement, avec la mâchoire, sur le film, et comportant, de plus, un dispositif-détecteur (32) disposé à l'avant ou à l'arrière de la cassette à film (12) et fournissant, pour la commande au moins de la tension (kv) du tube de la source de rayonnement 14), un signal électrique qui correspond au débit de dose d'un rayonnement X incident sur ledit dispositif-détecteur, caractérisé par le fait que l'unité d'entraînement en rotation (3) est susceptible d'être ajustée, à l'aide des dispositifs de déplacement (M1, M3), dans une position de départ (P), à partir de laquelle, et par réglage de l'unité d'entraînement en rotation (3) et la cassette à film (12) en correspondance avec la courbe, la cassette à film (12) est irradiée avec des rayons (51, 52) qui s'étendent en dehors de l'une (30a) des extrémités de la courbure (30) de la mâchoire, qu'à l'aide du dispositifdétecteur (32) la dose de rayonnement incidente est saisie, qu'un signal qui correspond à la dose du rayonnement est appliqué à une unité de calcul (34) dans laquelle on compare une grandeur qui correspond au signal avec une grandeur qui correspond à une valeur de dose prédéterminée, et qu'à partir de la comparaison on détermine les données de prise de vue (mA, kV, sec) qui sont nécessaires pour l'irradiation traversante de la tête du patient concerné, et avec lesquelles on commande le générateur de la source de rayonnement.

2. Appareil de radiodiagnostic selon la revendication 1, caractérisé par le fait qu'à l'unité de calcul (34) est reliée une mémoire (35) qui comporte un organe de mémorisation (36) qui fournit la grandeur de comparaison qui correspond à la valeur de dose prédéterminée.

3. Appareil de radiodiagnostic selon la revendication 2, caractérisé par le fait qu'il est prévu un organe de mémorisation (36) dont la grandeur de comparaison est réglable.

4. Appareil de radiodiagnostic selon la revendication 1, caractérisé par le fait que l'on détermine, à partir de la mesure de la dose au niveau du film, à l'aide du dispositif-détecteur (32), d'abord des grandeurs, spécifiques aux patients, pour la longueur de la mâchoire/largeur de la mâchoire, et que l'on détermine la position, spécifique au patient, de la couche à partir d'une comparaison de ces grandeurs avec des paires de valeurs prédéterminées pour des vitesses de film associées à certaines dimensions de mâchoire.

5. Appareil de radiodiagnostic selon la revendication 4, caractérisé par le fait que la durée entre le début de l'irradiation traversante de la tête du patient et le début de l'irradiation traversante de la mâchoire, est saisie par une unité de commande (33), que dans l'unité de calcul (34) on calcule, à partir du temps ($T_1$) déterminé par rapport à la durée totale (T) et à partir de la course ($L_G$) parcourue, pendant ce temps (T), sur le film (12), d'abord la course (a) sur le film jusqu'au début de l'irradiation traversante de la mâchoire, ensuite la longueur ($L_K$) de la mâchoire (30) qui est reproduite sur le film (12) et à partir de là, la grandeur pour la longueur de la mâchoire ($L_K$).

6. Appareil de radiodiagnostic selon la revendication 5, caractérisé par le fait que l'unité de calcul (34) comporte ou/est relié à une mémoire (35) dans laquelle sont contenus plusieurs valeurs de comparaison (37) pour différentes vitesses du film qui sont associées à des largeurs de mâchoires différentes, que la valeur, déterminée par l'unité de calcul (34), pour la largeur de la mâchoire spécifique au patient est comparée avec les valeurs contenues dans la mémoire et que l'on détermine, par la comparaison la position de la couche qui correspond à la constellation personnelle de la mâchoire du patient.

7. Appareil de radiodiagnostic selon l'une des revendications 1 à 6, caractérisé par le fait que le dispositif de déplacement comporte des premiers moyens de déplacement (14, 15, M1) à l'aide desquels le support (6) peut être entraîné en rotation autour d'un premier palier axial vertical (16), que des seconds moyens de déplacement (18 à 25, M2, M3) sont prévus, à l'aide desquels le support peut exécuter, pendant le mouvement de rotation autour du premier palier axial, un mouvement de basculement qui est transversal à l'axe de symétrie (30) de l'objet, le rayon de basculement (R) et le débattement (28) du mouvement de basculement étant choisis de telle façon que lors d'un déplacement de l'unité de rotation (6) il est toujours prévu une direction d'irradiation perpendiculaire à travers l'objet, pour une distance constante entre l'objet et le film, et qu'en outre la cassette à film (12) est montée dans son support (5) de façon à être déplaçable par rapport à la source de rayonnement (4), et qu'il est prévu une unité de commande (33) pour l'attaque de la transmission de déplacement (M1 à M4).

8. Appareil de radiodiagnostic selon la revendication 7, caractérisé par le fait que le support est réalisé, de préférence, sous la forme d'un anneau (6) fermé sur luimême qui est monté à rotation sur un élément de palier (7) qui est relié au chariot (2), que la cassette de film (12) est montée sur le support de cassette disposé sur l'anneau (6), de façon à pouvoir être déplacé par rapport à la source de rayonnement (4), et que les moyens (14, 15, M1; 18 à 25, M2, M3) du dispositif de déplacement et servant à déplacer l'anneau (6), d'une part, autour de son centre (16) et, d'autre part, autour d'un axe de basculement (26) disposé sur l'élément de palier (7) et relié au chariot (2), l'axe de basculement (26) étant pourvu d'un montage axial vertical.

9. Appareil de radiodiagnostic selon la revendication 8, caractérisé par le fait que l'élément de palier (7) est articulé au chariot (2) à l'aide de deux bras en ciseaux (24, 25) disposés à distance et de part et d'autre de l'axe de basculement (26), alors qu'entre les points d'articulation (21, 23; 20, 22) d'au moins un bras à ciseaux (24, 25), sont disposés les moyens de déplacement (M2, M3).

10. Appareil de radiodiagnostic selon la revendication 9, caractérisé par le fait qu'entre les points d'articulation (20, 23) des deux bras en ciseaux (24, 25) est prévu un mécanisme d'entraînement à broche, alors qu'à l'axe de basculement (26) est articulé une extrémité d'un bras télescopique (27) dont l'autre extrémité est fixée rigidement au chariot (2).

11. Appareil de radiodiagnostic selon l'une des revendications 1 à 3, caractérisé par le fait que pour le calcul de la valeur de dose qui représente la valeur de consigne, des moyens sont prévus qui définissent, d'une part, une valeur de seuil endessous de laquelle il n'est pas tenu compte de la dose mesurée par le dispositif-détecteur (32), et qui, par ailleurs, détermine une durée après laquelle est réalisée une mesure de répétition.

12. Appareil de radiodiagnostic selon l'une des revendications 1 à 11, caractérisé par le fait qu'il est prévu un dispositif-détecteur (32) du type optoélectronique, avec des éléments qui sont constitués par un cristal de scintillation, d'une part, et, d'autre part, par une photodiode.

**Claims**

1. Diagnostic dental x-ray apparatus for carrying out panoramic tomography of a patient's jaw having a rotary unit (3) which contains a support (6) for on the one hand a radiation source (4) and on the other hand a film cassette holder (5) for accepting a film cassette (12), the unit containing, furthermore adjustment devices (13, M1 to M3) with which on the one hand the support (6) is adjustable about vertical axes (16, 26) in an orbital curve corresponding to the dental arch and on the other hand the film cassette holder (5) or a film cassette (12, 50) insertable therein is adjustable in such a way that the teeth are successively imaged on the film together with the jaw, and containing, in addition, at least one detector arrangement (32) positioned either in front of or behind the film cassette (12), which arrangement supplies an electrical signal corresponding to the dose rate of x-ray radiation impinging thereon for the control of at least the tube voltage (kV) of the radiation source (4), characterised in that the rotary unit (3) can be adjusted into an initial position (P) by means of the adjustment devices (M1 to M3) from which, as the rotary unit (3) and film cassette (12) are being adjusted corresponding to the orbital curve, the film cassette (12) is acted upon by rays (S1, S2) which proceed outside the one end (30a) of the mandibular arch (30), in that the incoming radiation dose is detected by means of a detector arrangement (32), in that a signal corresponding to the radiation dose is supplied to a computation unit (34) in which a quantity corresponding to the signal is compared with a quantity corresponding to a prescribed dosage value, and in that the exposure data (mA, kV, sec) necessary for the transillumination of the relevant patient's head are determined from this comparison, with which exposure data the generator for the radiation source is controlled.

2. Diagnostic x-ray apparatus according to Claim 1, characterised in that a memory (35) containing a memory element (36) which supplies the comparison quantity corresponding to the prescribed dose value is connected to the computation unit (34).

3. Diagnostic x-ray apparatus according to Claim 2, characterised in that a memory element (36) adjustable in terms of its comparison quantity is provided.

4. Diagnostic x-ray apparatus according to Claim 1, characterised in that patient-specific quantities for jaw length/width are initially determined from the dosage measurement on the film by means of the detector arrangement (32) and the tomographic layer position specific to the patient can be established from a comparison of these quantities with prescribed value pairs for film speeds allocated to specific jaw dimensions.

5. Diagnostic x-ray apparatus according to Claim 4, characterised in that the time from the beginning of the transillumination of the patient's head up to the beginning of the transillumination of the jaw is detected by a control unit (33), in that in the computation unit (34), there is calculated from the ascertained time $(T_1)$ in relation to the total running time (T) and from the path distance $(L_G)$ covered in this time (T) on the film (12), initially the path distance (a) on the film up to the beginning of the transillumination of the jaw, then the length $(L_K)$ of the jaw (30) imaged on the film (12) and from this the quantity for the jaw length $(L_K)$ is calculated.

6. Diagnostic x-ray apparatus according to Claim 5, characterised in that the computation unit (34) contains a memory (35) or is connected to one, in which several comparison values (37) for film speeds allocated to different jaw widths are deposited, in that the value for the patient-specific jaw width detected by the computation unit (34) is compared with the deposited memory values and the layer position corresponding to the personal jaw configuration of the patient is determined from the comparison.

7. Diagnostic x-ray apparatus according to one of Claims 1 to 6, characterised in that adjustment device contains first adjustment means (14, 15, M1) by means of which the carrier (96) can be rotated about a first vertical axis (16), in that second adjustment means (18 to 25, M2, M3) are present by means of which the support, during the rotational movement, can be pivoted about the first axis at right angles to the symmetry axis (30) of the subject, the swivel radius (R) and the excursion (28) of the swivel motion being selected so that, given the movement of the rotary unit (6), a vertical transillumination through the subject is always provided given a constant interval from subject and film, in that furthermore the film cassette (12) is held in its holder (5) so that it can be adjusted relative to the radiation source (4) and in that a control unit (33) is present for controlling the adjustment drives (M1 to M4).

8. Diagnostic x-ray apparatus according to Claim 7, characterised in that the support is preferably constructed as a closed turntable ring (6) which is rotatable mounted on a bearing part (7) connected to the carriage (2), in that the film cassette (12) is held in the cassette holder (5) arranged on the ring (6) so that it can be adjusted relative to the radiation source (4) and in that the adjustment device contains means (14, 15, M1, 18 to 25, M2, M3) for adjusting the ring (6) on the one hand about its central point (16) and on the other hand about a swivel axis (26) with a vertical axis position, which swivel axis (26) is arranged on the bearing part (7) and is connected to the carriage (2).

9. Diagnostic x-ray apparatus according to Claim 8, characterised in that the bearing part (7) is hinged to the carriage (2) by means of two scissor arms (24, 25) arranged on both sides of the swivel axis (26) at a distance (a) and the adjustment means (M2, M3) are arranged between the hinge points (21, 23; 20, 22) of at least one scissor arm (24, 25).

10. Diagnostic x-ray apparatus according to Claim 9, characterised in that a spindle drive is provided between the hinge points (20, 23) of both scissor arms (24, 25), and one end of a telescopic arm (27) is hinged onto the swivel axis (26), the other end of said telescopic arm being rigidly fixed to the carriage (2).

11. Diagnostic x-ray apparatus according to one of Claims 1 to 3, characterised in that means is present for calculating the dose value representing one desired value, which means on the one hand defines a threshold value below which the dose measured by the detector arrangement (32) remains unconsidered and which on the other hand defines a time after whose expiration a repeated measurement is carried out.

12. Diagnostic x-ray apparatus according to one of Claims 1 to 11, characterised in that an electro-optic detector arrangement (32) is provided with elements which are constructed from a scintillation crystal on the one hand and a photodiode on the other.

FIG 1

1

FIG 2

FIG 3

FIG 4

FIG 5